# DEMANDE DE BREVET EUROPEEN

(11) **EP 2 881 095 A1**
(43) Date de publication de la demande: **10.06.2015**
(21) Numéro de dépôt: 14290384.8
(22) Date de dépôt: 04.12.2014
(51) Int. Cl.: A61F 13/14

(54) **DISPOSITIF DE CONTENTION POUR PREVENIR OU GUÉRIR TOUTES PATHOLOGIE ARTICULAIRE, MUSCULAIRE ET TENDINEUSE DU BASSIN**

(30) Priorité: 03.12.2013 FR 1302801
(71) Demandeur: Audran, Eric, 56200 La Gacilly (FR)
(72) Inventeur: La désignation de l'inventeur n'a pas encore été déposée

(57) **Abrégé**

La présente invention concerne un dispositif de contention pour la prévention ou la guérison pour toute pathologie musculaire, tendineuse ou articulaire de la cuisse et du bassin.

Il comprend, intégré dans un cuissard (1) une sangle supérieure non élastique (2) destiné à enserrer la taille et à être situé en dessous des deux crêtes iliaques et au moins une sangle inférieure non élastique (3) situé au niveau de la cuisse.

La tension des sangles est assurée par un système de boucle de serrage et de desserrage(4).

## Description

La présente invention concerne un dispositif de contention destiné à prévenir ou guérir toutes les pathologies musculaires, tendineuses et/ou articulaire de la cuisse et du bassin, notamment la pubalgie.

Une tendinite est une inflammation d'un tendon utilisé de manière excessive dans une mauvaise position articulaire le plus souvent et dans un déséquilibre musculaire notamment pour la pubalgie .Alors que la pathologie musculaire "physiologique" comme la contracture ou la pathologie musculaire avec lésion des fibres comme l'élongation ou le claquage n'entrainent pas d'inflammation articulaire mais ont besoin d'être "soutenu" mécaniquement afin d'éviter la récidive.

Le dispositif de la présente invention est utile pour tous les sportifs pratiquant des courses rapides avec changement de direction comme le football, le hand ball, le hockey, le rugby etc ... ces sportifs souffrent surtout de tendinites des muscles du membre inférieur avec pour première place les adducteurs.

Le traitement thérapeutique classique des tendinites est une prescription médicale d'anti-inflammatoires, des séances de kinésithérapie et un arrêt de la compétition et de l'entrainement ainsi qu'un traitement thérapeutique complémentaire comme l'ostéopathie, la posturologie ou la podologie.

L'inconvénient principal de cette thérapeutique est sa durée importante et le risque de récidive.

Un sportif de haut niveau ou un sportif tout court simplement motivé va vite s'impatienter si la durée d'arrêt sportif est longue .En outre, l'arrêt de l'entrainement chez un sportif de haut niveau peut être préjudiciable à sa carrière sportive.

La présente invention a pour but de remédier aux inconvénients précités et de fournir un dispositif permettant de soigner et/ou de prévenir : les douleurs articulaires et tendineuses inflammatoires comme la pubalgie et les douleurs musculaires avec ou sans lésions musculaires.

Ceci permettant une poursuite ou une reprise rapide de l'activité physique afin de ne pas perdre le "capital compétitif' du sportif.

La cause principale de la pubalgie est l'hyper mobilité du pubis liée à:
1: un verrouillage pubien défaillant
2: un manque de mobilité de la hanche

### 1: La symphyse pubienne est le siège de mouvement de faible amplitude .Une hyper mobilité peut entrainer une pubalgie .Le dispositif de la présente invention va avoir son efficacité, sur le maintien relatif de cette symphyse pubienne.

Le complexe abdominaux-pubo-fémoral croisé(APFC) met en jeu les abdominaux et les adducteurs .Leurs fibres se confondent en avant du pubis constituant une continuité fonctionnelle croisée responsable du verrouillage pubien.

Le complexe APFC implique des muscles adducteurs et abdominaux qui peuvent être en déséquilibre:
Adducteurs puissants rétractés et abdominaux faibles (footballeur) ou
Abdominaux puissants et adducteurs faibles (rugbyman ou hockeyeur)

Tout déséquilibre de force musculaire entre abdominaux et adducteurs favorise une défaillance du verrouillage pubien.

Toute défaillance du verrouillage pubien se traduit par une hyper mobilité du pubis (torsion, compression, cisaillement)

Les contraintes appliquées au pubis sont majorées en appui uni -podal, les contraintes appliquées sur le pubis sont convergentes, lors des déplacements multidirectionnels : saut, arrêt brusque, changement de direction rapide alors qu'en appui bi-podal, les forces résultantes des contraintes appliquées au pubis sont divergentes donnant une meilleure répartition des charges.

### 2 : Le bon fonctionnement du complexe lombo pelvien fémoral dépend du bon équilibre entre les groupes musculaires antérieurs et postérieurs du bassin: les fléchisseurs de la hanche : muscle droit antérieur, psoas, sartorius et le tenseur du fascia lata. Les extenseurs de la hanche : muscle grand fessier profond et grand fessier superficiel

La pubalgie est le plus souvent secondaire à un déséquilibre de force au bénéfice des fléchisseurs de hanche .Ce déséquilibre réduit la mobilité de hanche en extension et entraine une hyper mobilité compensatrice du bassin qui favorise une hyper mobilité des articulations sacro- iliaques et du pubis en torsion.

Le dispositif de contention pour la prévention de toutes pathologies musculaires, tendineuses et articulaires des parties moyennes ou supérieures de la cuisse et du bassin comprend, intégré dans un cuissard, une sangle supérieure non élastique destinée à enserrer la taille et à être en dessous des 2 crêtes iliaques et au moins une sangle inférieure non élastique situé au niveau de la cuisse. La tension des sangles étant assurée par un système de boucle de serrage et de desserrage.

Selon un mode de réalisation particulier, le dispositif pour la prévention ou la guérison de la pubalgie comporte une sangle supérieure et deux sangles inférieures à savoir une sangle sur chaque cuisse. La sangle supérieure est située en dessous des deux crêtes iliaques alors que les sangles inférieures sont situées au niveau interne de la cuisse à la jonction de la symphyse pubienne et des tendons des muscles adducteurs et, au niveau externe de la cuisse en dessous du relief du grand trochanter de la hanche.

Pour le traitement d'une pathologie musculaire de la cuisse, la sangle inférieure enserre la cuisse juste au niveau de la pathologie de façon à maintenir une compression suffisante sur le muscle à protéger.

Les sangles sont non élastique d'une largeur de l'ordre de 2 à 3 cm et de préférence en tissu. Le réglage de la tension des sangles est assuré par une boucle de serrage et de desserrage. Les sangles inférieures sont intégrées dans un cuissard comprenant une échancrure facilitant leur déplacement vertical.

La tension des sangles doit être forte pour avoir une efficacité maximum, l'objectif étant de maintenir le bassin avec la sangle supérieure et d'absorber les contraintes mécaniques montantes des membres inférieures avec les sangles inférieures.

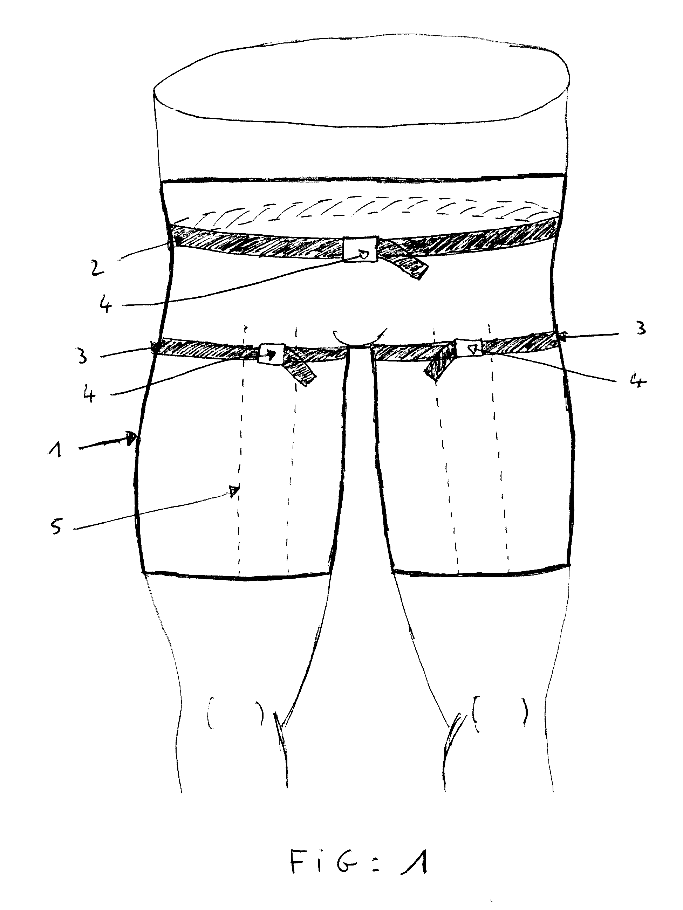

La figure 1 est une vue du dispositif traitant la pubalgie.

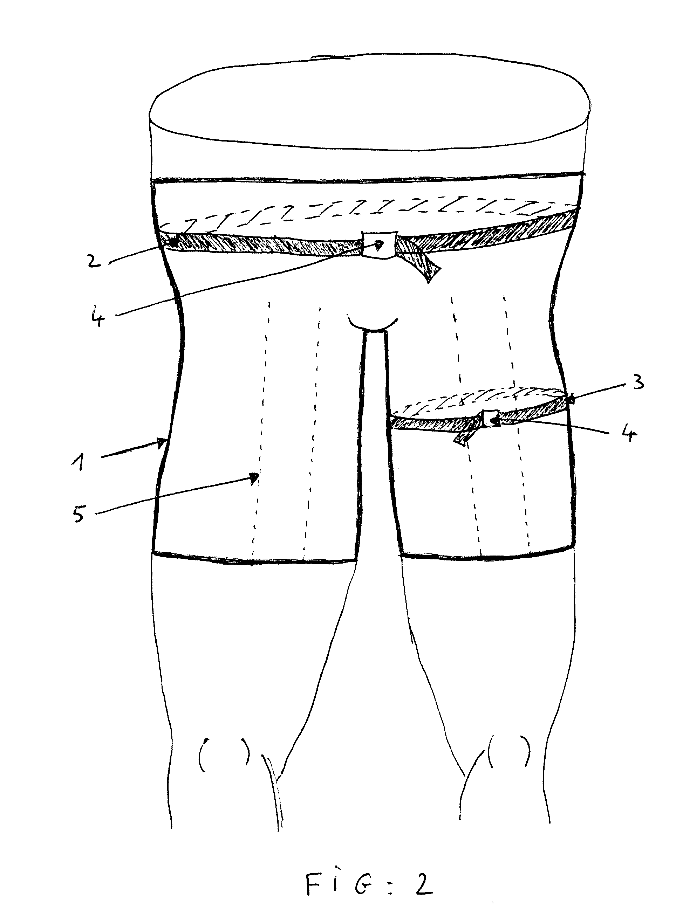

La figure 2 est une vue du dispositif traitant une pathologie musculaire et tendineuse.

En référence à ces figures, le cuissard (1) comprend dans sa partie supérieure une sangle (2) non élastique de préférence en tissu d'une largeur de l'ordre de 2 à 3 cms, cette sangle est pourvue d'une boucle de serrage et de desserrage (4), elle est située en dessous des crêtes iliaques. Les sangles (3) placées sur la cuisse sont également non élastiques de préférence en tissu, d'une largeur identique. Elles sont placées selon la pathologie c'est à dire à la jonction entre la symphyse pubienne et les tendons des muscles adducteurs s'il s'agit de la pubalgie ou au niveau de la contracture ou de l'élongation pour la pathologie musculaire .Les sangles (3) sont intégrées dans une échancrure qui facilite leur positionnement adéquat.

## Revendications

1. Dispositif de contention pour la prévention ou la guérison de toute pathologie musculaire , tendineuses et/ou articulaire de la partie moyenne et supérieure de la cuisse et du bassin **caractérisé en ce qu'**il comprend , intégré dans un cuissard (1), une sangle supérieure non élastique (2) destinée à enserrer la taille et à être située en dessous des deux crêtes iliaques et au moins une sangle inférieure non élastique (3) située au niveau de la cuisse , **caractérisée en ce qu'**il comprend la tension des sangles étant assurée par un système de boucle de serrage et de desserrage(4).

2. Dispositif de contention selon la revendication 1 **caractérisé en ce qu'**il comprend, pour la prévention ou la guérison de la pubalgie, une sangle supérieure (2) et deux sangles inférieures (3), une sangle sur chaque cuisse.

3. Dispositif de contention selon l'une ou l'autre des revendications 1 et 2 **caractérisées en ce que** les sangles non élastiques (2) et (3) sont de préférence en tissu et ont une largeur de l'ordre de deux à trois cms.

4. Dispositif de contention selon l'une ou l'autre des revendications 1 à 3 **caractérisées en ce que** le réglage de la tension des sangles (2) et (3) est assuré par une boucle de serrage et de desserrage (4).

5. Dispositif de contention selon l'une ou l'autre des revendications 1 à 4 **caractérisées en ce que** les sangles (2) et (3) sont intégrées dans un cuissard et les sangles (3) sont intégrés dans un cuissard comprenant une échancrure (5) facilitant leur déplacement vertical.
